# FASCICULE DE BREVET EUROPEEN

(11) **EP 4 326 185 B1**
(45) Date de publication et mention de la délivrance du brevet: **29.05.2024**
(21) Numéro de dépôt: 23732127.8
(22) Date de dépôt: 12.06.2023
(51) Int. Cl.: A61B 90/16, A61C 1/08, A61C 17/10, A61C 19/04

(54) **BLOCS, SYSTÈMES ET PROCÉDÉS DE STABILISATION DENTAIRE**
BLÖCKE, SYSTEME UND VERFAHREN ZUR ZAHNSTABILISIERUNG
BLOCKS, SYSTEMS AND METHODS FOR DENTAL STABILISATION

(30) Priorité: 12.07.2022 FR 2207123
(43) Date de publication de la demande: 28.02.2024
(73) Titulaire: Digicuto, 13008 Marseille (FR)
(72) Inventeur: Koubi Stefan, 13008 Marseille (FR); Gurel Galip, 13008 Marseille (FR); Tourbah Karim, 13008 Marseille (FR); Pinta Maxence, 13008 Marseille (FR)
(74) Mandataire: A.P.I. Conseil
(86) Numéro de dépôt international: PCT/EP2023/065691
(87) Numéro de publication internationale: WO 2024/012781

(56) Documents cités:
- WO-A1-2017/212406
- WO-A1-2020/214697
- US-A1- 2010 291 503
- US-A1- 2018 125 349

## Description

### Domaine technique

L'invention concerne le domaine des interventions dentaires. En particulier, elle concerne des blocs de stabilisation dentaire, des systèmes de stabilisation dentaire et des procédés de stabilisation dentaire pour maintenir la bouche du patient ouverte tout en empêchant les dents de se rencontrer.

Des dispositifs similaires sont connus du document US2018/125349A1.

### Technique antérieure

Dans le domaine dentaire, on connait des dispositifs de stabilisation dentaires (« bite blocks », en anglais) que l'on désigne également par les termes suivants : « support de mâchoire », « bloc de mordu », « pièce de morsure » ou « bloc de morsure ».

Toutefois, les dispositifs de stabilisation dentaires connus permettent au patient de facilement en desserrer ses dents.

Or, dans le cadre de certaines interventions dentaires, il est primordial que le patient ne desserre pas ses dents du dispositif de stabilisation dentaire.

Ainsi, il existe un besoin pour une solution qui permet d'éviter la situation décrite ci-dessus.

### Résumé de l'invention

L'invention vise à résoudre, au moins partiellement, ce besoin.

L'invention est définie dans les revendications 1 et 12 ci-dessous. Un premier aspect de l'invention vise un bloc de stabilisation dentaire qui est destiné à être mordu, d'un seul côté de la bouche, entre les molaires et/ou prémolaires supérieures et inférieures d'un patient, dites dents, pour maintenir la bouche du patient ouverte pendant une intervention dentaire de manière à empêcher les dents de se rencontrer, autorisant ainsi l'accès à des dents et/ou des gencives du côté opposé de la bouche et à l'avant de la bouche, sans être gêné sensiblement par le bloc de stabilisation dentaire.

Le bloc de stabilisation dentaire comprend :
- un corps, de forme essentiellement parallélépipédique, qui comprend, lorsque le bloc de stabilisation dentaire est en place dans la bouche du patient,
- une paroi supérieure, présentant une surface externe supérieure de serrage qui est conçue pour entrer en contact avec les dents supérieures, la surface externe supérieure de serrage comprenant au moins un premier orifice de sortie,
- une paroi inférieure, présentant une surface externe inférieure de serrage qui est conçue pour entrer en contact avec les dents inférieures, la surface externe inférieure de serrage comprenant au moins un deuxième orifice de sortie,

- une première paroi latérale qui est orientée vers l'intérieur de la bouche,
- une deuxième paroi latérale qui est orientée vers une face interne de joue,
- une paroi d'extrémité avant qui est orientée vers l'avant de la bouche,
- une paroi d'extrémité arrière qui est orientée vers l'arrière de la bouche,
- au moins un premier canal interne qui débouche vers l'extérieur du corps au niveau d'au moins le premier orifice de sortie et/ou au moins le deuxième orifice de sortie, le premier canal interne définissant un chemin d'écoulement qui est configuré pour être parcouru par un matériau d'enregistrement d'occlusion destiné à durcir et à être moulé au moins sur les dents,
- au moins un deuxième canal interne qui débouche vers l'extérieur du corps au niveau d'un orifice d'entrée qui est configuré pour recevoir au moins une portion d'extrémité d'un dispositif d'injection du matériau d'enregistrement d'occlusion, l'orifice d'entrée étant disposé sur une surface externe de la première paroi latérale et/ou la paroi d'extrémité avant, et
- une première région interne de jonction qui est configurée pour établir une communication fluidique entre le premier canal interne et le deuxième canal interne.

Dans un premier mode de réalisation, le bloc de stabilisation dentaire comprend en outre,
- une pluralité de premiers canaux internes qui est agencée de sorte que, en réponse à l'injection du matériau d'enregistrement d'occlusion, le matériau d'enregistrement d'occlusion arrive sensiblement en même temps au niveau des orifices de sorties, et
- une pluralité de deuxièmes régions internes de jonction qui est agencée pour que chaque deuxième région interne de jonction établisse une communication fluidique entre au moins deux premiers canaux internes.

Dans un deuxième mode de réalisation, le matériau d'enregistrement d'occlusion est choisi parmi : des cires, des pâtes d'oxyde de zinc-eugénol, des élastomères silicones par addition, des élastomères polyesthers et toutes combinaisons de ceux-ci.

Dans un troisième mode de réalisation, la première paroi latérale et la deuxième paroi latérale sont conçues pour s'étendre chacune vers le haut et/ou vers le bas de la bouche au-delà de la surface externe supérieure de serrage et/ou de la surface externe inférieure de serrage pour recouvrir les dents supérieures et/ou inférieures de manière à former une enceinte supérieure et/ou une enceinte inférieure de section en forme de U dans lesquelles le matériau d'enregistrement d'occlusion et des dents supérieures et/ou inférieures peuvent être retenus, la base de la section en forme de U de l'enceinte supérieure ou de l'enceinte inférieure contenant, respectivement, la surface externe supérieure de serrage ou la surface externe inférieure de serrage, les ailes de la section en forme de U de l'enceinte supérieure ou de l'enceinte inférieure contenant, respectivement, la première paroi latérale et la deuxième paroi latérale.

Dans un quatrième mode de réalisation, la deuxième paroi latérale est pourvue d'une partie d'écarteur de joue qui est destinée à écarter la face interne de joue de la deuxième paroi latérale, la partie d'écarteur de joue faisant saillie depuis la deuxième paroi latérale vers la face interne de joue, et, lorsque le bloc de stabilisation dentaire est en place dans la bouche du patient, présente une forme de L avec un coude arrondi, dont l'une des branches, dite branche d'appui, et le coude arrondi prennent appui sur la face interne de joue et dont l'autre branche, dite branche en porte-à-faux, est en porte-à-faux et prend appui, au moins en partie, sur la paroi d'extrémité arrière.

Dans une mise en oeuvre du quatrième mode de réalisation, la partie d'écarteur de joue comprend au moins un troisième canal interne qui est agencé, au moins en partie, à l'intérieur du coude arrondi, le troisième canal interne débouchant vers l'extérieur du corps de la branche d'appui au niveau d'un orifice d'aspiration et au niveau d'un orifice d'évacuation, l'orifice d'aspiration étant disposé sur la branche en porte-à-faux, l'orifice d'évacuation étant disposé sur la branche d'appui.

Dans un exemple de la mise en oeuvre du quatrième mode de réalisation, la branche d'appui comprend une partie de guidage de canule d'aspiration qui est ouverte en direction de la deuxième paroi latérale et qui s'étend axialement depuis l'orifice d'évacuation jusqu'à l'extrémité libre de la branche d'appui.

Dans un cinquième mode de réalisation, la surface externe supérieure de serrage et la surface externe inférieure de serrage sont pourvues d'une pluralité de nervures de perturbation d'écoulement de fluide.

Dans un sixième mode de réalisation, lorsqu'il dépend du quatrième mode de réalisation, le bloc de stabilisation dentaire comprend, en outre, une branche d'extension qui s'étend de façon oblique ou perpendiculaire depuis l'extrémité libre de la branche d'appui vers l'extérieur de la bouche, la branche d'extension étant conçue pour former une région de changement de direction qui, lorsque la branche d'extension dévie pour sortir de la bouche du patient, présente une surface extérieure qui recouvre la commissure labiale de la bouche,
dans lequel,
au moins une région d'extrémité libre de la branche d'extension comprend au moins un élément de support qui est adapté pour supporter au moins un élément de référence de position qui est conçu pour servir d'élément de guidage d'un système de navigation pour chirurgie dentaire, l'élément de référence de position étant adapté pour, lorsque l'élément de référence de position est déplacé en réponse à au moins un mouvement de la tête du patient, déclencher un recalage d'une ou plusieurs images dentaires associées au patient, en fonction du mouvement de la tête du patient.

L'invention vise aussi un système de stabilisation dentaire comprenant,
- un bloc de stabilisation dentaire selon le premier aspect de l'invention, et
- un élément de référence de position qui est configuré pour être couplé à au moins un élément de support de la région d'extrémité libre de la branche d'extension, l'élément de référence de position étant conçu pour servir d'élément de guidage d'un système de navigation pour chirurgie dentaire, l'élément de référence de position étant adapté pour, lorsque l'élément de référence de position est déplacé en réponse à au moins un mouvement de la tête du patient, déclencher un recalage d'une ou plusieurs images dentaires associées au patient, en fonction du mouvement de la tête du patient.

Dans un premier mode de réalisation, le système de stabilisation dentaire comprend :
- deux blocs de stabilisation dentaire selon le premier aspect de l'invention, les blocs de stabilisation dentaire étant configurés pour être positionnés sur des côtés opposés de la bouche du patient.

L'invention vise enfin un procédé de stabilisation dentaire pendant une intervention dentaire.

Le procédé comprend :
- une étape d'installation, dans la bouche du patient, d'au moins un bloc de stabilisation dentaire selon le premier aspect de l'invention, sur des molaires et/ou les prémolaires supérieures et inférieures d'un patient, dites dents,
- une étape d'introduction d'une portion d'extrémité d'un dispositif d'injection d'un matériau d'enregistrement d'occlusion jusqu'à l'orifice d'entrée, le matériau d'enregistrement d'occlusion étant destiné à durcir et à être moulé au moins sur les dents, et
- une étape d'injection du matériau d'enregistrement d'occlusion dans l'orifice d'entrée.

### Brève description des dessins

D'autres caractéristiques et avantages de l'invention seront mieux compris à la lecture de la description qui va suivre et en référence aux dessins annexés, donnés à titre illustratif et nullement limitatif.
[Fig.1] La [Fig.1] représente une première vue en perspective d'un bloc de stabilisation dentaire selon l'invention.
[Fig.2] La [Fig.2] représente le bloc de stabilisation dentaire de la [Fig.1], lorsqu'il est en place dans la bouche d'un patient.
[Fig.3] La [Fig.3] représente une deuxième vue en perspective d'un bloc de stabilisation dentaire selon l'invention.
[Fig.4] La [Fig.4] représente une vue agrandie de la [Fig.1].
[Fig.5] La [Fig.5] représente une première vue en coupe d'un bloc de stabilisation dentaire selon l'invention.
[Fig.6] La [Fig.6] représente une vue agrandie de la [Fig.3].
[Fig.7] La [Fig.7] représente une vue de dessus de la [Fig.3].
[Fig.8] La [Fig.8] représente une deuxième vue en coupe d'un bloc de stabilisation dentaire selon l'invention.
[Fig.9] La [Fig.9] représente une vue de dessus d'un mode de réalisation de l'invention.
[Fig.10] La [Fig.10] représente un procédé selon l'invention.

### Description des modes de réalisation

L'un des objectifs de cette invention est de fournir un dispositif de stabilisation dentaire qui empêche un patient, lors d'une intervention dentaire, de facilement en desserrer ses dents.

Pour cela, l'inventeur propose un bloc de stabilisation dentaire qui comprend des canaux internes qui débouchent sur les faces du bloc de stabilisation dentaire qui entrent en contact avec les dents du patient. Ces canaux internes sont connectés à une entrée par laquelle on peut injecter un matériau d'enregistrement d'occlusion qui est destiné à durcir et à être moulé sur les dents du patient.

De cette manière, lorsque le matériau d'enregistrement d'occlusion a durci et s'est moulé autour des dents, il devient plus difficile pour le patient de desserrer ses dents du bloc de stabilisation dentaire.

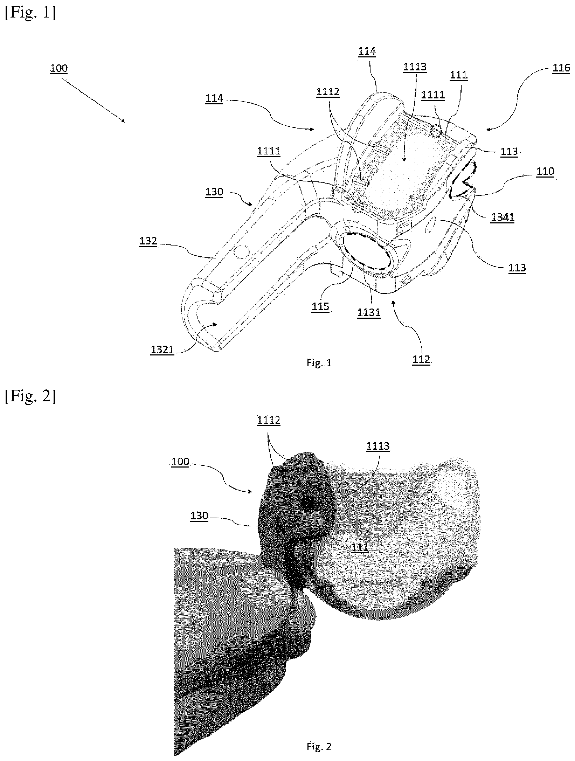

Ainsi, comme illustré sur la [Fig.1], l'invention concerne un bloc de stabilisation dentaire 100.

En pratique, le bloc de stabilisation dentaire 100 est destiné à être mordu, d'un seul côté de la bouche, entre les molaires et/ou prémolaires supérieures et inférieures d'un patient, appelées ci-après « dents ».

Dit autrement, lorsque le bloc de stabilisation dentaire 100 est en place dans la bouche du patient, le patient peut mordre le bloc de stabilisation dentaire 100, soit seulement avec ses molaires supérieures et inférieures, soit seulement avec ses prémolaires supérieures et inférieures ou soit à la fois avec ses molaires supérieures et inférieures et prémolaires supérieures et inférieures.

Dans un mode de réalisation privilégié de l'invention, on pourra prévoir différentes tailles du bloc de stabilisation dentaire 100 en fonction de la morphologie dentaire du patient, à savoir la disposition de ses dents sur la mâchoire supérieure et sur la mâchoire inférieure.

Par exemple, on pourra prévoir un modèle pour les enfants et un modèle pour les adultes.

Toutefois, selon les besoins, on pourra envisager d'autres agencements de prise en compte de la morphologie dentaire des patients, et ce, sans nécessiter de modifications substantielles de l'invention.

La [Fig.2] illustre le bloc de stabilisation dentaire 100 lorsqu'on le positionne sur des dents inférieures situées dans le côté gauche de la bouche du patient.

En pratique, le bloc de stabilisation dentaire 100 permet de maintenir la bouche du patient ouverte pendant une intervention dentaire de manière à empêcher les dents de se rencontrer.

Ainsi, le bloc de stabilisation dentaire 100 autorise l'accès à des dents et des gencives situées du côté opposé de la bouche et à l'avant de la bouche, et ce, sans être gêné sensiblement par le bloc de stabilisation dentaire 100.

Autrement dit, lorsque le bloc de stabilisation dentaire 100 est en place dans la bouche du patient, le praticien qui réalise l'intervention dentaire peut accéder, à l'intérieur de la bouche, aux dents et aux gencives qui se trouvent dans la partie avant de la bouche ainsi que la partie de la bouche qui est située à l'opposé de la position du bloc de stabilisation dentaire 100.

Ainsi, dans un premier exemple, si on positionne le bloc de stabilisation dentaire 100 sur des dents situées dans le côté droit de la bouche du patient, alors le praticien peut accéder aux dents et gencives qui sont disposées à l'avant de la bouche ainsi qu'à celles qui sont disposées à gauche de la bouche.

De même, dans un deuxième exemple, si on positionne le bloc de stabilisation dentaire 100 sur des dents situées dans le côté gauche de la bouche du patient, alors le praticien peut accéder aux dents et gencives qui sont disposées à l'avant de la bouche ainsi qu'à celles qui sont disposées à droite de la bouche.

De retour à l'invention, le bloc de stabilisation dentaire 100 comprend un corps 110 qui est de forme essentiellement parallélépipédique.

Ainsi, le corps 110 présente une forme de parallélépipède, à savoir, la forme d'un polyèdre à six faces dont les six faces sont parallèles deux à deux.

En pratique, la forme du corps 110 est sensiblement incurvée pour prendre en compte la courbe approximative des dents supérieures et inférieures dans une mâchoire humaine.

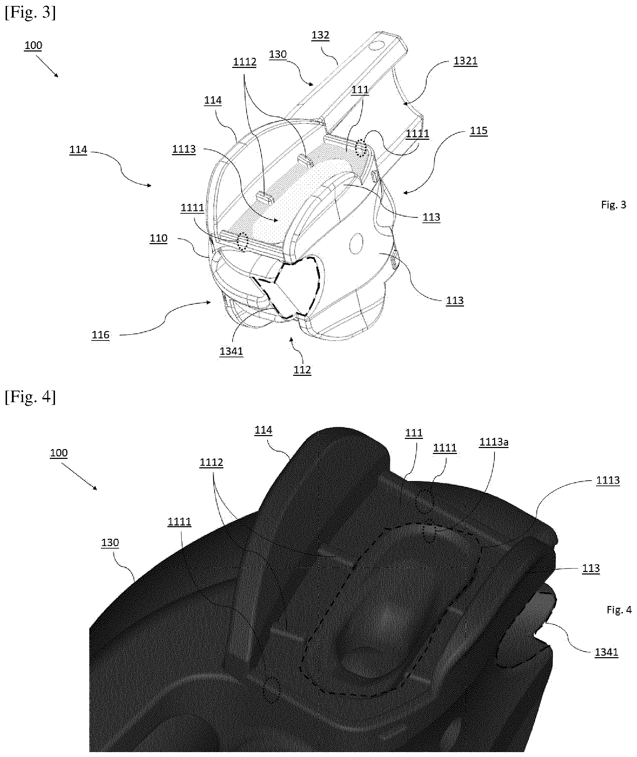

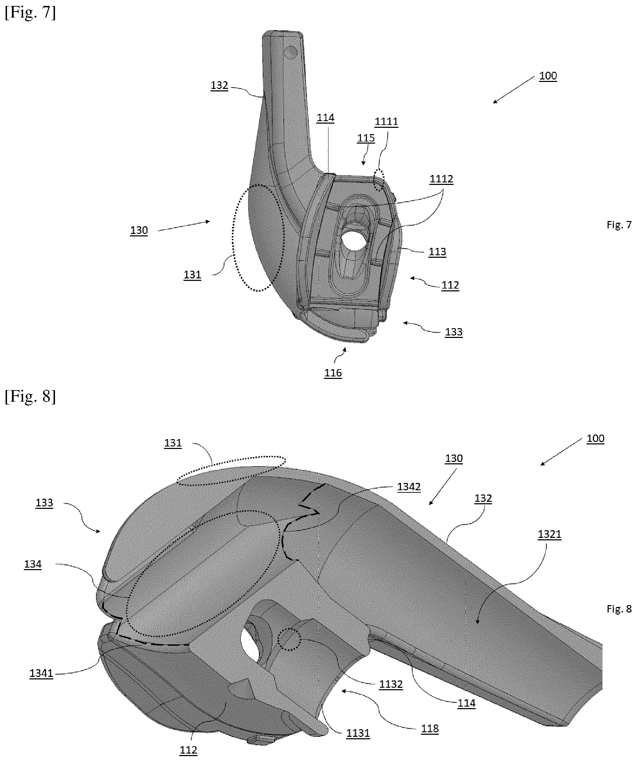

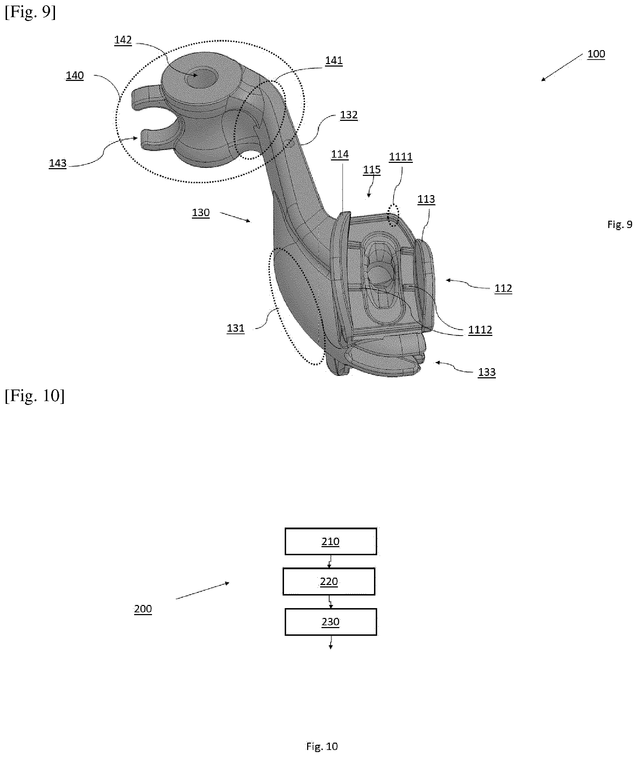

En particulier, comme illustré sur la [Fig.1], la [Fig.3], la [Fig.7] et la [Fig.9], lorsque le bloc de stabilisation dentaire 100 est en place dans la bouche du patient, le corps 110 comprend une paroi supérieure 111, une paroi inférieure 112, une première paroi latérale 113, une deuxième paroi latérale 114, une paroi d'extrémité avant 115 et une paroi d'extrémité arrière 116.

En pratique, la paroi supérieure 111 présente une surface externe supérieure de serrage qui est conçue pour entrer en contact avec les dents supérieures.

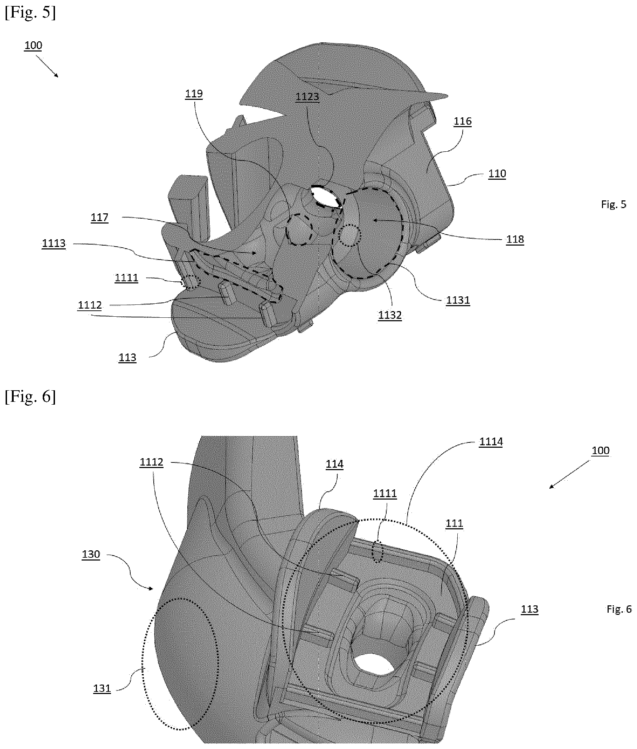

Dans une mise en oeuvre particulière de la surface externe supérieure de serrage, comme illustré sur la [Fig.1], la [Fig.3], la [Fig.4], la [Fig.5], la [Fig.6], la [Fig.7] et la [Fig.9], la surface externe supérieure de serrage comprend un rebord périphérique supérieur 1111.

En particulier, le rebord périphérique supérieur 1111 est configuré pour retenir un matériau d'enregistrement d'occlusion tel que décrit ci-après.

Dans une mise en oeuvre particulière de la surface externe supérieure de serrage, comme illustré sur la [Fig.1], la [Fig.3], la [Fig.4], la [Fig.5], la [Fig.6], la [Fig.7] et la [Fig.9], celle-ci est pourvue d'une pluralité de nervures de perturbation d'écoulement de fluide 1112.

En pratique, chaque nervure de perturbation d'écoulement de fluide 1112 est configurée pour, lorsque le bloc de stabilisation dentaire 100 est en place dans la bouche du patient, occasionner une perturbation de l'écoulement d'un fluide qui s'écoule sur la surface externe supérieure de serrage.

Dans un premier exemple de cette mise en oeuvre particulière, les nervures de perturbation d'écoulement de fluide 1112 s'étendent vers le haut à partir de la surface externe supérieure de serrage.

Dans un deuxième exemple de cette mise en oeuvre particulière, les nervures de perturbation d'écoulement de fluide 1112 s'étendent à travers un axe d'écoulement de fluide de la surface externe supérieure de serrage.

Dans un troisième exemple de cette mise en oeuvre particulière, les nervures de perturbation d'écoulement de fluide 1112 présentent une surface extérieure supérieure de turbulence qui est choisie parmi : une surface étagée, une surface striée, une surface alvéolée ou une surface rugueuse.

En outre, comme illustré sur la [Fig.1], la [Fig.3], la [Fig.4] et la [Fig.5], la surface externe supérieure de serrage comprend au moins un premier orifice de sortie 1113.

Dans une mise en oeuvre particulière du premier orifice de sortie 1113, comme illustré sur la [Fig.4], le premier orifice de sortie 1113 comprend un décrochement intérieur périphérique 1113a de manière à former au moins une première surface de décrochage périphérique qui est agencée en dessous de la surface externe supérieure de serrage.

De manière similaire à la paroi supérieure 111, la paroi inférieure 112 présente une surface externe inférieure de serrage qui est conçue pour entrer en contact avec les dents inférieures.

Dans une mise en oeuvre particulière de la surface externe inférieure de serrage, celle-ci est pourvue d'une pluralité de nervures de perturbation d'écoulement de fluide qui sont similaires aux nervures de perturbation d'écoulement de fluide 1112 de la surface externe supérieure de serrage.

Dans un premier exemple de cette mise en oeuvre particulière, les nervures de perturbation d'écoulement de fluide s'étendent vers le bas à partir de la surface externe inférieure de serrage.

Dans un deuxième exemple de cette mise en oeuvre particulière, les nervures de perturbation d'écoulement de fluide s'étendent à travers un axe d'écoulement de fluide de la surface externe inférieure de serrage.

Dans un troisième exemple de cette mise en oeuvre particulière, les nervures de perturbation d'écoulement de fluide présentent une surface extérieure inférieure de turbulence qui est choisie parmi : une surface étagée, une surface striée, une surface alvéolée ou une surface rugueuse.

En outre, la surface externe inférieure de serrage comprend au moins un deuxième orifice de sortie 1123.

Comme illustré sur la [Fig.5], de manière similaire au premier orifice de sortie 1113, dans une mise en oeuvre particulière du deuxième orifice de sortie 1123, celui-ci comprend un décrochement intérieur périphérique de manière à former au moins une deuxième surface de décrochage périphérique qui est agencée en dessous de la surface externe inférieure de serrage.

Comme illustré sur la [Fig.2], en lien avec la [Fig.1], la [Fig.3], la [Fig.4], la [Fig.5], la [Fig.6], la [Fig.7] et la [Fig.9], lorsque le bloc de stabilisation dentaire 100 est en place dans la bouche du patient :
- la première paroi latérale 113 est orientée vers l'intérieur de la bouche,
- la deuxième paroi latérale 114 est orientée vers une face interne de joue, en particulier la face interne de joue située du côté de la bouche où le bloc de stabilisation dentaire 100 est installé. Ainsi, si on installe le bloc de stabilisation dentaire 100 dans le côté droit de la bouche du patient, alors la deuxième paroi latérale 114 est orientée vers la face interne de joue droite du patient. Par ailleurs, si on installe le bloc de stabilisation dentaire 100 dans le côté gauche de la bouche du patient, alors la deuxième paroi latérale 114 est orientée vers la face interne de joue gauche du patient,
- la paroi d'extrémité avant 115 est orientée vers l'avant de la bouche, et
- la paroi d'extrémité arrière 116 est orientée vers l'arrière de la bouche.

En outre, comme illustré sur la [Fig.5], le corps 110 comprend au moins un premier canal interne 117, au moins un deuxième canal interne 118 et une première région interne de jonction 119.

En particulier, le premier canal interne 117, qui se trouve à l'intérieur du corps 110, débouche vers l'extérieur du corps 110 au niveau d'au moins un premier orifice de sortie 1113 et/ou au moins un deuxième orifice de sortie 1123.

Dit autrement, on peut faire déboucher le premier canal interne 117 vers l'extérieur, soit en passant seulement par le premier orifice de sortie 1113, soit en passant seulement par le deuxième orifice de sortie 1123 ou soit en passant à la fois par le premier orifice de sortie 1113 et le deuxième orifice de sortie 1123.

Par ailleurs, le premier canal interne 117 définit un chemin d'écoulement qui est configuré pour être parcouru par un matériau d'enregistrement d'occlusion destiné à durcir et à être moulé au moins sur les dents.

Dans une première mise en oeuvre du matériau d'enregistrement d'occlusion, celui-ci est choisi parmi des cires.

Par exemple, on pourra choisir parmi les cires minérales (p. ex. des alcanes comme les Paraffines), les cires d'origine naturelle (p. ex. des esters comme les cires de Carnauba, les cires d'abeilles), les cires d'origines synthétiques (p. ex. les cires de polyéthylènes, de polyéthylène glycol, cires d'hydrocarbures hydrogénés ou halogénés) et toutes combinaisons de celles-ci.

Dans une deuxième mise en oeuvre du matériau d'enregistrement d'occlusion, celui-ci est choisi parmi des pâtes d'oxyde de zinc-eugénol.

Dans une troisième mise en oeuvre du matériau d'enregistrement d'occlusion, celui-ci est choisi parmi des élastomères silicones par addition.

Dans une quatrième mise en oeuvre du matériau d'enregistrement d'occlusion, celui-ci est choisi parmi des élastomères polyesthers.

Toutefois, selon les besoins, on pourra envisager d'utiliser d'autres matériaux dentaires qui sont capables de se déformer sous contrainte occlusale, d'enregistrer cette déformation et de la restituer sans modifications, et ce, sans nécessiter de modifications substantielles de l'invention.

Le deuxième canal interne 118, qui se trouve à l'intérieur du corps 110, débouche vers l'extérieur du corps 110 au niveau d'un orifice d'entrée 1131.

L'orifice d'entrée 1131 est configuré pour recevoir au moins une portion d'extrémité d'un dispositif d'injection du matériau d'enregistrement d'occlusion.

Dans un exemple, la portion d'extrémité selon l'invention est un embout mélangeur d'un injecteur d'enregistrement d'occlusion de type connu.

Dans une mise en oeuvre particulière de l'orifice d'entrée 1131, comme illustré sur la [Fig.5] et la [Fig.8], l'orifice d'entrée 1131 comprend un épaulement 1132 contre lequel l'extrémité du dispositif d'injection du matériau d'enregistrement d'occlusion peut venir en butée.

En outre, dans l'invention, l'orifice d'entrée 1131 est disposé sur une surface externe de la première paroi latérale 113 et/ou la paroi d'extrémité avant 115.

Autrement dit, on peut disposer l'orifice d'entrée 1131, soit seulement sur une surface externe de la première paroi latérale 113, soit seulement sur une surface externe de la paroi d'extrémité avant 115 ou soit à la fois sur une surface externe de la première paroi latérale 113 et sur une surface externe de la paroi d'extrémité avant 115.

Enfin, la première région interne de jonction 119 est configurée pour établir une communication fluidique entre le premier canal interne 117 et le deuxième canal interne 118.

Ainsi, lorsque l'on injecte le matériau d'enregistrement d'occlusion dans l'orifice d'entrée 1131, le matériau d'enregistrement d'occlusion peut sortir au niveau des orifices de sorties 1113, 1123.

La [Fig.5] illustre les orifices de sorties 1113, 1123.

Dans une première façon de réaliser l'invention, le corps 110 comprend une pluralité de premiers canaux internes 117 et une pluralité de deuxièmes régions internes de jonction.

En pratique, la pluralité de premiers canaux internes 117, qui se trouve à l'intérieur du corps 110, est agencée de sorte que, en réponse à l'injection du matériau d'enregistrement d'occlusion, le matériau d'enregistrement d'occlusion arrive sensiblement en même temps au niveau des orifices de sorties 1113, 1123.

En outre, la pluralité de deuxièmes régions internes de jonction est agencée pour que chaque deuxième région interne de jonction établisse une communication fluidique entre au moins deux premiers canaux internes 117.

En d'autres termes, chaque deuxième région interne de jonction peut établir une connexion fluidique entre deux, trois, quatre ou plus de quatre premiers canaux internes 117.

Dans une deuxième façon de réaliser l'invention, la première paroi latérale 113 et la deuxième paroi latérale 114 sont conçues pour s'étendre chacune vers le haut et/ou vers le bas de la bouche au-delà de la surface externe supérieure de serrage et/ou de la surface externe inférieure de serrage.

Dit autrement, dans la deuxième façon de réaliser l'invention, la première paroi latérale 113 et la deuxième paroi latérale 114 s'étendent, soit seulement vers le haut de la bouche, soit seulement vers le bas de la bouche ou soit à la fois vers le haut et vers le bas de la bouche.

Cet agencement est tel qu'il permet, lorsque le bloc de stabilisation dentaire 100 est en place dans la bouche du patient, de recouvrir les dents supérieures et/ou inférieures de manière à former une enceinte supérieure et/ou une enceinte inférieure de section en forme de U dans lesquelles le matériau d'enregistrement d'occlusion et des dents supérieures et/ou inférieures peuvent être retenus.

En d'autres termes, lorsque la première paroi latérale 113 et la deuxième paroi latérale 114 s'étendent seulement vers le haut de la bouche, elles forment l'enceinte supérieure. En outre, lorsque la première paroi latérale 113 et la deuxième paroi latérale 114 s'étendent seulement vers le bas de la bouche, elles forment l'enceinte inférieure. Enfin, lorsque la première paroi latérale 113 et la deuxième paroi latérale 114 s'étendent à la fois vers le haut et le bas de la bouche, elles forment l'enceinte supérieure et l'enceinte inférieure.

En particulier, comme illustré sur la [Fig.6], dans l'enceinte supérieure 1114 et dans l'enceinte inférieure, la base de la section en forme de U de l'enceinte supérieure 1114 ou de l'enceinte inférieure contient, respectivement, la surface externe supérieure de serrage ou la surface externe inférieure de serrage.

En outre, les ailes de la section en forme de U de l'enceinte supérieure 1114 ou de l'enceinte inférieure contiennent, respectivement, la première paroi latérale 113 et la deuxième paroi latérale 114.

Ainsi, l'enceinte supérieure 1114 présente une section en forme de U, dont la base contient la surface externe supérieure de serrage et dont les ailes contiennent la première paroi latérale 113 et la deuxième paroi latérale 114.

Par ailleurs, de façon similaire à l'enceinte supérieure 1114, l'enceinte inférieure présente une section en forme de U, dont la base contient la surface externe inférieure de serrage et dont les ailes contiennent la première paroi latérale 113 et la deuxième paroi latérale 114.

Dans un mode de réalisation particulier, comme illustré sur la [Fig.1], la [Fig.3], la [Fig.4] et la [Fig.6], la deuxième paroi latérale 114 s'étend plus haut que la première paroi latérale 113.

Dans une troisième façon de réaliser l'invention, la deuxième paroi latérale 114 est pourvue d'une partie d'écarteur de joue 130 qui est destinée à écarter la face interne de joue de la deuxième paroi latérale 114.

En particulier, comme illustré sur la [Fig.1], la [Fig.2], la [Fig.3], la [Fig.6], la [Fig.7] , la [Fig.8] et la [Fig.9], la partie d'écarteur de joue 130 fait saillie depuis la deuxième paroi latérale 114 vers la face interne de joue.

En outre, comme illustré sur la [Fig.7] et la [Fig.9], lorsque le bloc de stabilisation dentaire 100 est en place dans la bouche du patient, la partie d'écarteur de joue 130 présente une forme de L avec un coude arrondi 131.

En pratique, comme illustré sur la [Fig.7] et la [Fig.9], l'une des branches de la partie d'écarteur de joue 130, appelée ci-après «branche d'appui » 132 et le coude arrondi 131 prennent appui sur la face interne de joue.

Par ailleurs, l'autre branche de la partie d'écarteur de joue 130, appelée ci-après « branche en porte-à-faux » 133 est en porte-à-faux et prend appui, au moins en partie, sur la paroi d'extrémité arrière 116.

Dans un premier mode de réalisation de la troisième façon de réaliser l'invention, comme illustré sur la [Fig.8], la partie d'écarteur de joue 130 comprend au moins un troisième canal interne 134 qui est agencé, au moins en partie, à l'intérieur du coude arrondi 131.

Dans un premier mode de réalisation du premier mode de réalisation de la troisième façon de réaliser l'invention, le canal interne 134 est agencé à l'intérieur du coude arrondi 131 et à l'intérieur de la branche d'appui 132.

Dans un deuxième mode de réalisation du premier mode de réalisation de la troisième façon de réaliser l'invention, le canal interne 134 est agencé à l'intérieur du coude arrondi 131 et à l'intérieur de la branche en porte-à-faux 133.

Dans un troisième mode de réalisation du premier mode de réalisation de la troisième façon de réaliser l'invention, le canal interne 134 est agencé à l'intérieur du coude arrondi 131, à l'intérieur de la branche d'appui 132 et à l'intérieur de la branche en porte-à-faux 133.

En pratique, le troisième canal interne 134 débouche vers l'extérieur du corps de la branche d'appui 132 au niveau d'un orifice d'aspiration 1341 et au niveau d'un orifice d'évacuation 1342.

En particulier, comme illustré sur la [Fig.8], l'orifice d'aspiration 1341 est disposé sur la branche en porte-à-faux 133 en amont de la paroi d'extrémité arrière 116 par rapport à la bouche du patient.

Par ailleurs, l'orifice d'évacuation 1342 est disposé sur la branche d'appui 132 en aval de l'orifice d'aspiration 1341 par rapport à la bouche du patient.

Dans une mise en oeuvre particulière du premier mode de réalisation de la troisième façon de réaliser l'invention, comme illustré sur la [Fig.8], la branche d'appui 132 comprend une partie de guidage de canule d'aspiration 1321 qui est ouverte en direction de la deuxième paroi latérale 114 et qui s'étend axialement depuis l'orifice d'évacuation 1342 jusqu'à l'extrémité libre de la branche d'appui 132.

Dans un deuxième mode de réalisation de la troisième façon de réaliser l'invention, comme illustré sur la [Fig.9], le bloc de stabilisation dentaire 100 comprend, en outre, une branche d'extension 140 qui s'étend de façon oblique ou perpendiculaire depuis l'extrémité libre de la branche d'appui 132 vers l'extérieur de la bouche.

En pratique, la branche d'extension 140 est conçue pour former une région de changement de direction 141 qui, lorsque la branche d'extension 140 dévie pour sortir de la bouche du patient, présente une surface extérieure qui recouvre la commissure labiale de la bouche.

Par ailleurs, au moins une région d'extrémité libre de la branche d'extension 140 comprend au moins un élément de support 142, 143 qui est adapté pour supporter au moins un élément de référence de position.

Dans l'invention, l'élément de référence de position est conçu pour servir d'élément de guidage d'un système de navigation pour chirurgie dentaire de type connu.

On entend par « système de navigation pour chirurgie dentaire », un système de navigation assisté par ordinateur qui permet de fournir une assistance visuelle à un praticien lors d'une intervention dentaire. Plusieurs technologies de guidage existent (p. ex. par fluoroscopie, optique ou électromagnétique), mais le but reste le même, à savoir de fournir à l'aide de l'imagerie médicale un retour précis de la position du bloc de stabilisation dentaire 100.

En pratique, l'élément de référence de position est adapté pour, lorsque l'élément de référence de position est déplacé en réponse à au moins un mouvement de la tête du patient, déclencher un recalage d'une ou plusieurs images dentaires associées au patient, en fonction du mouvement de la tête du patient.

L'invention couvre également un premier système de stabilisation dentaire.

En pratique, le premier système de stabilisation dentaire comprend le bloc de stabilisation dentaire 100 selon le deuxième mode de réalisation de la troisième façon de réaliser l'invention et un élément de référence de position tel que décrit ci-dessus.

En particulier, l'élément de référence de position est configuré pour être couplé à au moins un élément de support 142, 143 de la région d'extrémité libre de la branche d'extension 140.

L'invention couvre aussi un deuxième système de stabilisation dentaire.

En pratique, le deuxième système de stabilisation dentaire comprend deux blocs de stabilisation dentaire 100 selon l'une quelconque des variantes de l'invention.

En particulier, les deux blocs de stabilisation dentaire 100 du deuxième système de stabilisation dentaire sont configurés pour être positionnés sur des côtés opposés de la bouche du patient.

Enfin, comme illustré sur la [Fig.10], l'invention couvre un procédé 200 de stabilisation dentaire pendant une intervention dentaire.

Tout d'abord, le procédé 200 comprend une étape d'installation 210, dans la bouche du patient, d'au moins un bloc de stabilisation dentaire 100 selon l'une quelconque des variantes de l'invention, sur des molaires et/ou les prémolaires supérieures et inférieures d'un patient, appelées ci-après « dents »,.

Ensuite, le procédé 200 comprend une étape d'introduction 220 d'une portion d'extrémité d'un dispositif d'injection d'un matériau d'enregistrement d'occlusion jusqu'à l'orifice d'entrée 1131, le matériau d'enregistrement d'occlusion étant destiné à durcir et à être moulé au moins sur les dents.

Enfin, le procédé 200 comprend une étape d'injection 230 du matériau d'enregistrement d'occlusion dans l'orifice d'entrée 1131.

## Revendications

1. Bloc de stabilisation dentaire (100) destiné à être mordu, d'un seul côté de la bouche, entre les molaires et/ou prémolaires supérieures et inférieures d'un patient, dites dents, pour maintenir la bouche du patient ouverte pendant une intervention dentaire de manière à empêcher les dents de se rencontrer, autorisant ainsi l'accès à des dents et/ou des gencives du côté opposé de la bouche et à l'avant de la bouche, sans être gêné sensiblement par le bloc de stabilisation dentaire (100), le bloc de stabilisation dentaire (100) comprenant :
- un corps (110), de forme essentiellement parallélépipédique, qui comprend, lorsque le bloc de stabilisation dentaire (100) est en place dans la bouche du patient,
- une paroi supérieure (111), présentant une surface externe supérieure de serrage qui est conçue pour entrer en contact avec les dents supérieures, la surface externe supérieure de serrage comprenant au moins un premier orifice de sortie (1113),
- une paroi inférieure (112), présentant une surface externe inférieure de serrage qui est conçue pour entrer en contact avec les dents inférieures, la surface externe inférieure de serrage comprenant au moins un deuxième orifice de sortie (1123),
- une première paroi latérale (113) qui est orientée vers l'intérieur de la bouche,
- une deuxième paroi latérale (114) qui est orientée vers une face interne de joue,
- une paroi d'extrémité avant (115) qui est orientée vers l'avant de la bouche,
- une paroi d'extrémité arrière (116) qui est orientée vers l'arrière de la bouche,
- au moins un premier canal interne (117) qui débouche vers l'extérieur du corps (110) au niveau d'au moins le premier orifice de sortie (1113) et/ou au moins le deuxième orifice de sortie (1123), le premier canal interne (117) définissant un chemin d'écoulement qui est configuré pour être parcouru par un matériau d'enregistrement d'occlusion destiné à durcir et à être moulé au moins sur les dents,
- au moins un deuxième canal interne (118) qui débouche vers l'extérieur du corps (110) au niveau d'un orifice d'entrée (1131) qui est configuré pour recevoir au moins une portion d'extrémité d'un dispositif d'injection du matériau d'enregistrement d'occlusion, l'orifice d'entrée (1131) étant disposé sur une surface externe de la première paroi latérale (113) et/ou la paroi d'extrémité avant (115), et
- une première région interne de jonction (119) qui est configurée pour établir une communication fluidique entre le premier canal interne (117) et le deuxième canal interne (118).

2. Bloc de stabilisation dentaire (100) selon la revendication 1, comprenant en outre,
- une pluralité de premiers canaux internes (117) qui est agencée de sorte que, en réponse à l'injection du matériau d'enregistrement d'occlusion, le matériau d'enregistrement d'occlusion arrive sensiblement en même temps au niveau des orifices de sorties (1113, 1123), et
- une pluralité de deuxièmes régions internes de jonction qui est agencée pour que chaque deuxième région interne de jonction établisse une communication fluidique entre au moins deux premiers canaux internes (117).

3. Bloc de stabilisation dentaire (100) selon l'une quelconque des revendications 1 à 2, dans lequel le matériau d'enregistrement d'occlusion est choisi parmi : des cires, des pâtes d'oxyde de zinc-eugénol, des élastomères silicones par addition, des élastomères polyesthers et toutes combinaisons de ceux-ci.

4. Bloc de stabilisation dentaire (100) selon l'une quelconque des revendications 1 à 3, dans lequel la première paroi latérale (113) et la deuxième paroi latérale (114) sont conçues pour s'étendre chacune vers le haut et/ou vers le bas de la bouche au-delà de la surface externe supérieure de serrage et/ou de la surface externe inférieure de serrage pour recouvrir les dents supérieures et/ou inférieures de manière à former une enceinte supérieure et/ou une enceinte inférieure de section en forme de U dans lesquelles le matériau d'enregistrement d'occlusion et des dents supérieures et/ou inférieures peuvent être retenus, la base de la section en forme de U de l'enceinte supérieure ou de l'enceinte inférieure contenant, respectivement, la surface externe supérieure de serrage ou la surface externe inférieure de serrage, les ailes de la section en forme de U de l'enceinte supérieure ou de l'enceinte inférieure contenant, respectivement, la première paroi latérale (113) et la deuxième paroi latérale (114).

5. Bloc de stabilisation dentaire (100) selon l'une quelconque des revendications 1 à 4, dans lequel la deuxième paroi latérale (114) est pourvue d'une partie d'écarteur de joue (130) qui est destinée à écarter la face interne de joue de la deuxième paroi latérale (114), la partie d'écarteur de joue (130) faisant saillie depuis la deuxième paroi latérale (114) vers la face interne de joue, et, lorsque le bloc de stabilisation dentaire (100) est en place dans la bouche du patient, présente une forme de L avec un coude arrondi (131), dont l'une des branches, dite branche d'appui (132), et le coude arrondi (131) prennent appui sur la face interne de joue et dont l'autre branche, dite branche en porte-à-faux (133), est en porte-à-faux et prend appui, au moins en partie, sur la paroi d'extrémité arrière (116).

6. Bloc de stabilisation dentaire (100) selon la revendication 5, dans lequel la partie d'écarteur de joue (130) comprend au moins un troisième canal interne (134) qui est agencé, au moins en partie, à l'intérieur du coude arrondi (131), le troisième canal interne (134) débouchant vers l'extérieur du corps de la branche d'appui (132) au niveau d'un orifice d'aspiration (1341) et au niveau d'un orifice d'évacuation (1342), l'orifice d'aspiration (1341) étant disposé sur la branche en porte-à-faux (133), l'orifice d'évacuation (1342) étant disposé sur la branche d'appui (132).

7. Bloc de stabilisation dentaire (100) selon la revendication 6, dans lequel la branche d'appui (132) comprenant une partie de guidage de canule d'aspiration (1321) qui est ouverte en direction de la deuxième paroi latérale (114) et qui s'étend axialement depuis l'orifice d'évacuation (1342) jusqu'à l'extrémité libre de la branche d'appui (132).

8. Bloc de stabilisation dentaire (100) selon l'une quelconque des revendications 1 à 7, dans lequel la surface externe supérieure de serrage et la surface externe inférieure de serrage sont pourvues d'une pluralité de nervures de perturbation d'écoulement de fluide (1112).

9. Bloc de stabilisation dentaire (100) selon l'une quelconque des revendications 5 à 7, comprenant, en outre, une branche d'extension (140) qui s'étend de façon oblique ou perpendiculaire depuis l'extrémité libre de la branche d'appui (132) vers l'extérieur de la bouche, la branche d'extension (140) étant conçue pour former une région de changement de direction (141) qui, lorsque la branche d'extension (140) dévie pour sortir de la bouche du patient, présente une surface extérieure qui recouvre la commissure labiale de la bouche,
dans lequel,
au moins une région d'extrémité libre de la branche d'extension (140) comprend au moins un élément de support (142, 143) qui est adapté pour supporter au moins un élément de référence de position qui est conçu pour servir d'élément de guidage d'un système de navigation pour chirurgie dentaire, l'élément de référence de position étant adapté pour, lorsque l'élément de référence de position est déplacé en réponse à au moins un mouvement de la tête du patient, déclencher un recalage d'une ou plusieurs images dentaires associées au patient, en fonction du mouvement de la tête du patient.

10. Système de stabilisation dentaire comprenant,
- un bloc de stabilisation dentaire (100) selon la revendication 9, et
- un élément de référence de position qui est configuré pour être couplé à au moins un élément de support (142, 143) de la région d'extrémité libre de la branche d'extension (140), l'élément de référence de position étant conçu pour servir d'élément de guidage d'un système de navigation pour chirurgie dentaire, l'élément de référence de position étant adapté pour, lorsque l'élément de référence de position est déplacé en réponse à au moins un mouvement de la tête du patient, déclencher un recalage d'une ou plusieurs images dentaires associées au patient, en fonction du mouvement de la tête du patient.

11. Système de stabilisation dentaire comprenant :
- deux blocs de stabilisation dentaire (100) selon l'une quelconque des revendications 1 à 9, les blocs de stabilisation dentaire (100) étant configurés pour être positionnés sur des côtés opposés de la bouche du patient.

12. Procédé (200) de stabilisation dentaire pendant une intervention dentaire, le procédé comprenant :
- une étape d'installation (210), dans la bouche du patient, d'au moins un bloc de stabilisation dentaire (100) selon l'une quelconque des revendications 1 à 9, sur des molaires et/ou les prémolaires supérieures et inférieures d'un patient, dites dents,
- une étape d'introduction (220) d'une portion d'extrémité d'un dispositif d'injection d'un matériau d'enregistrement d'occlusion jusqu'à l'orifice d'entrée (1131), le matériau d'enregistrement d'occlusion étant destiné à durcir et à être moulé au moins sur les dents, et
- une étape d'injection (230) du matériau d'enregistrement d'occlusion dans l'orifice d'entrée (1131).

## Patentansprüche

1. Block zur Zahnstabilisierung (100), der bestimmt ist, auf einer einzigen Seite des Mundes zwischen den oberen und unteren Molaren und/oder Prämolaren eines Patienten, bezeichnet als Zähne, gebissen zu werden, um den Mund des Patienten während einer Zahnbehandlung derart geöffnet zu halten, dass vermieden wird, dass sich die Zähne begegnen, wodurch der Zugriff auf die Zähne und/oder das Zahnfleisch auf der gegenüberliegenden Seite des Mundes und im vorderen Mund gestattet ist, ohne durch den Block zur Zahnstabilisierung (100) erheblich behindert zu werden, wobei der Block zur Zahnstabilisierung (100) umfasst:
- einen im Wesentlichen parallelepipedischen Körper (110), der umfasst, wenn der Block zur Zahnstabilisierung (100) im Mund des Patienten platziert ist,
- eine obere Wand (111), die eine obere äußere Spannoberfläche aufweist, die eingerichtet ist, um mit den oberen Zähnen in Kontakt zu treten, wobei die obere äußere Spannoberfläche mindestens eine erste Ausgangsöffnung (1113) umfasst,
- eine untere Wand (112), die eine untere äußere Spannoberfläche aufweist, die eingerichtet ist, um mit den unteren Zähnen in Kontakt zu treten, wobei die untere äußere Spannoberfläche mindestens eine zweite Ausgangsöffnung (1123) umfasst,
- eine erste Seitenwand (113), die in das Innere des Mundes ausgerichtet ist,
- eine zweite Seitenwand (114), die zu einer Innenfläche der Wange ausgerichtet ist,
- eine vordere Endwand (115), die zur Vorderseite des Mundes ausgerichtet ist,
- eine hintere Endwand (116), die zur Hinterseite des Mundes ausgerichtet ist,
- mindestens einen ersten inneren Kanal (117), der nach außerhalb des Körpers (110) im Bereich von mindestens der ersten Ausgangsöffnung (1113) und/oder mindestens der zweiten Ausgangsöffnung (1123) ausmündet, wobei der erste innere Kanal (117) einen Fließweg definiert, der dazu ausgelegt ist, von einem Okklusionsregistrierungsmaterial durchquert zu werden, das zum Härten und zum Formen mindestens auf den Zähnen bestimmt ist,
- mindestens einen zweiten inneren Kanal (118), der nach außerhalb des Körpers (110) im Bereich einer Eingangsöffnung (1131) ausmündet, der dazu ausgelegt ist, mindestens einen Endabschnitt einer Injektionsvorrichtung des Okklusionsregistrierungsmaterials aufzunehmen, wobei die Eingangsöffnung (1131) auf einer äußeren Oberfläche der ersten Seitenwand (113) und/oder der vorderen Endwand (115) angeordnet ist, und
- eine erste innere Verbindungsregion (119), die dazu ausgelegt ist, eine Fluidkommunikation zwischen dem ersten inneren Kanal (117) und dem zweiten inneren Kanal (118) herzustellen.

2. Block zur Zahnstabilisierung (100) nach Anspruch 1, umfassend ferner
- eine Vielzahl erster innerer Kanäle (117), die derart ausgebildet sind, dass als Antwort auf die Injektion des Okklusionsregistrierungsmaterials das Okklusionsregistrierungsmaterial etwa gleichzeitig im Bereich der Ausgangsöffnungen (1113, 1123) ankommt, und
- eine Vielzahl zweiter innerer Verbindungsregionen, die ausgebildet sind, damit jede zweite innere Verbindungsregion eine Fluidkommunikation zwischen mindestens zwei ersten inneren Kanälen (117) herstellt.

3. Block zur Zahnstabilisierung (100) nach einem der Ansprüche 1 bis 2, wobei das Okklusionsregistrierungsmaterial aus den Wachsen, den Zinkoxid-Eugenol-Pasten, den Additions-Silikon-Elastomeren, den Polyester-Elastomeren und allen Kombinationen derselben ausgewählt ist.

4. Block zur Zahnstabilisierung (100) nach einem der Ansprüche 1 bis 3, wobei die erste Seitenwand (113) und die zweite Seitenwand (114) ausgebildet sind, um sich jeweils nach oben und/oder nach unten im Mund über die obere äußere Spannoberfläche und/oder die untere äußere Spannoberfläche hinaus zu erstrecken, um die oberen und/oder unteren Zähne derart zu bedecken, dass eine obere Einschließung und/oder eine untere Einschließung mit einem U-förmigen Querschnitt gebildet wird, in denen das Okklusionsregistrierungsmaterial und obere und/oder untere Zähne gehalten werden können, wobei die Basis des U-förmigen Querschnitts der oberen Einschließung oder der unteren Einschließung jeweils die obere äußere Spannoberfläche oder die untere äußere Spannoberfläche enthält, wobei die Flügel des U-förmigen Querschnitts der oberen Einschließung oder der unteren Einschließung jeweils die erste Seitenwand (113) und die zweite Seitenwand (114) enthalten.

5. Block zur Zahnstabilisierung (100) nach einem der Ansprüche 1 bis 4, wobei die zweite Seitenwand (114) mit einem Wangenabstandshalterteil (130) versehen ist, der dazu bestimmt ist, die Wangeninnenfläche von der zweiten Seitenwand (114) zu beabstanden, wobei der Wangenabstandshalterteil (130) aus der zweiten Seitenwand (114) zur Wangeninnenfläche hervorsteht, und, wenn der Block zur Zahnstabilisierung (100) im Mund des Patienten platziert ist, eine L-Form mit einem abgerundeten Winkel (131) aufweist, von dem einer der Schenkel, bezeichnet als Stützschenkel (132), und der abgerundete Winkel (131) auf der Wangeninnenfläche aufliegen und dessen anderer Schenkel, bezeichnet als Überhangschenkel (133), überhängt und mindestens zum Teil auf der hinteren Endwand (116) aufliegt.

6. Block zur Zahnstabilisierung (100) nach Anspruch 5, wobei der Wangenabstandshalterteil (130) mindestens einen dritten inneren Kanal (134) umfasst, der mindestens zum Teil im Inneren des abgerundeten Winkels (131) eingerichtet ist, wobei der dritte innere Kanal (134) nach außerhalb des Körpers des Stützschenkels (132) im Bereich einer Ansaugöffnung (1341) und im Bereich einer Ableitungsöffnung (1342) ausmündet, wobei die Ansaugöffnung (1341) auf dem Überhangschenkel (133) angeordnet ist, wobei die Ableitungsöffnung (1342) auf dem Stützschenkel (132) angeordnet ist.

7. Block zur Zahnstabilisierung (100) nach Anspruch 6, wobei der Stützschenkel (132) einen Ansaugkanülen-Führungsteil (1321) umfasst, der in Richtung der zweiten Seitenwand (114) offen ist und der sich axial ab der Ableitungsöffnung (1342) bis zum freien Ende des Stützschenkels (132) erstreckt.

8. Block zur Zahnstabilisierung (100) nach einem der Ansprüche 1 bis 7, wobei die obere äußere Spannoberfläche und die untere äußere Spannoberfläche mit einer Vielzahl von Fluidflussstörungsrippen (1112) versehen sind.

9. Block zur Zahnstabilisierung (100) nach einem der Ansprüche 5 bis 7, umfassend ferner einen Erweiterungsschenkel (140), der sich schräg oder senkrecht ab dem freien Ende des Stützschenkels (132) nach außerhalb des Mundes erstreckt, wobei der Erweiterungsschenkel (140) ausgebildet ist, um eine Richtungswechselregion (141) zu bilden, die, wenn der Erweiterungsschenkel (140) ausweicht, um aus dem Mund des Patienten auszutreten, eine äußere Oberfläche aufweist, die den labialen Mundwinkel des Mundes bedeckt,
wobei
mindestens eine freie Endregion des Erweiterungsschenkels (140) mindestens ein Stützelement (142, 143) umfasst, das zum Stützen von mindestens einem Positionsreferenzelement geeignet ist, das ausgebildet ist, um als Führungselement eines Navigationssystems für Zahnchirurgie zu dienen, wobei das Positionsreferenzelement geeignet ist, um, wenn das Positionsreferenzelement als Antwort auf mindestens eine Bewegung des Kopfes des Patienten verschoben ist, eine Neukalibrierung von einem oder mehreren Zahnbildern, die dem Patienten zugeordnet sind, in Abhängigkeit von der Bewegung des Kopfes des Patienten auszulösen.

10. System zur Zahnstabilisierung, umfassend
- einen Block zur Zahnstabilisierung (100) nach Anspruch 9, und
- ein Positionsreferenzelement, das dazu ausgelegt ist, mit mindestens einem Stützelement (142, 143) der freien Endregion des Erweiterungsschenkels (140) verbunden zu sein, wobei das Positionsreferenzelement ausgebildet ist, um als Führungselement eines Navigationssystems für Zahnchirurgie zu dienen, wobei das Positionsreferenzelement geeignet ist, um, wenn das Positionsreferenzelement als Antwort auf mindestens eine Bewegung des Kopfes des Patienten verschoben ist, eine Neukalibrierung von einem oder mehreren Zahnbildern, die dem Patienten zugeordnet sind, in Abhängigkeit von der Bewegung des Kopfes des Patienten auszulösen.

11. System zur Zahnstabilisierung, umfassend:
- zwei Blöcke zur Zahnstabilisierung (100) nach einem der Ansprüche 1 bis 9, wobei die Blöcke zur Zahnstabilisierung (100) dazu ausgelegt sind, auf gegenüberliegenden Seiten des Mundes des Patienten positioniert zu sein.

12. Verfahren (200) zu Zahnstabilisierung während einer Zahnbehandlung, wobei das Verfahren umfasst:
- einen Schritt des Installierens (210) im Mund des Patienten mindestens eines Blocks zur Zahnstabilisierung (100) nach einem der Ansprüche 1 bis 9 auf den oberen und unteren Molaren und/oder Prämolaren eines Patienten, bezeichnet als Zähne,
- einen Schritt des Einführens (220) eines Endabschnitts einer Injektionsvorrichtung eines Okklusionsregistrierungsmaterial bis zur Eingangsöffnung (1131), wobei das Okklusionsregistrierungsmaterial bestimmt ist, zu härten und mindestens auf den Zähnen geformt zu werden, und
- einen Schritt des Injizierens (230) des Okklusionsregistrierungsmaterials in die Eingangsöffnung (1131).

## Claims

1. A dental stabilization block (100) intended to be bitten, only on one side of the mouth, between the upper and lower molars and/or premolars of a patient, called teeth, to keep the patient's mouth open during a dental procedure so as to prevent the teeth from meeting, thereby authorizing access to teeth and/or gums on the opposite side of the mouth and at the front of the mouth, without being substantially hampered by the dental stabilization block (100), the dental stabilization block (100) comprising:
- a body (110), of essentially parallelepiped shape, which comprises, when the dental stabilization block (100) is in place in the patient's mouth,
- an upper wall (111), having an upper outer tightening surface which is designed to come into contact with the upper teeth, the upper outer tightening surface comprising at least a first outlet orifice (1113),
- a lower wall (112), having a lower outer tightening surface which is designed to come into contact with the lower teeth, the lower outer tightening surface comprising at least a second outlet orifice (1123),
- a first side wall (113) which is oriented towards the inside of the mouth,
- a second side wall (114) which is oriented towards an inner face of the cheek,
- a front end wall (115) which is oriented towards the front of the mouth,
- a rear end wall (116) which is oriented towards the back of the mouth,
- at least a first inner channel (117) which opens out towards the outside of the body (110) at the level of at least the first outlet orifice (1113) and/or at least the second outlet orifice (1123), the first inner channel (117) defining a flow path which is configured to be traversed by a bite registration material intended to harden and to be molded at least on the teeth,
- at least a second inner channel (118) which opens out towards the outside of the body (110) at the level of an inlet orifice (1131) which is configured to receive at least one end portion of a device for injecting the bite registration material, the inlet orifice (1131) being disposed on an outer surface of the first side wall (113) and/or the front end wall (115), and
- a first inner junction region (119) which is configured to establish fluid communication between the first inner channel (117) and the second inner channel (118) .

2. The dental stabilization block (100) according to claim 1, further comprising,
- a plurality of first inner channels (117) which is arranged so that, in response to the injection of the bite registration material, the bite registration material arrives substantially at the same time at the level of the outlet orifices (1113, 1123), and
- a plurality of second inner junction regions which is arranged so that each second inner junction region establishes fluid communication between at least two first inner channels (117).

3. The dental stabilization block (100) according to any one of claims 1 to 2, wherein the bite registration material is chosen from: waxes, zinc oxide-eugenol pastes, addition silicone elastomers, polyester elastomers and any combinations thereof.

4. The dental stabilization block (100) according to any one of claims 1 to 3, wherein the first side wall (113) and the second side wall (114) are designed to each extend towards the top and/or towards the bottom of the mouth beyond the upper outer tightening surface and/or the lower outer tightening surface to cover the upper and/or lower teeth so as to form an upper enclosure and/or a lower enclosure of U-shaped section in which the bite registration material and upper and/or lower teeth can be retained, the base of the U-shaped section of the upper enclosure or of the lower enclosure containing, respectively, the upper outer tightening surface or the lower outer tightening surface, the wings of the U-shaped section of the upper enclosure or of the lower enclosure containing, respectively, the first side wall (113) and the second side wall (114).

5. The dental stabilization block (100) according to any one of claims 1 to 4, wherein the second side wall (114) is provided with a cheek retractor part (130) which is intended to retract the inner cheek face of the second side wall (114), the cheek retractor portion (130) protruding from the second side wall (114) towards the inner cheek face and, when the dental stabilization block (100) is in place in the patient's mouth, has an L shape with a rounded bend (131), whose one of the branches, called bearing branch (132), and the rounded bend (131) bear on the inner cheek face and whose other branch, called cantilever branch (133), is cantilevered and bears, at least partly, on the rear end wall (116).

6. The dental stabilization block (100) according to claim 5, wherein the cheek retractor part (130) comprises at least a third inner channel (134) which is arranged, at least partly, inside the rounded bend (131), the third inner channel (134) opening out towards the outside of the body of the bearing branch (132) at the level of a suction orifice (1341) and at the level of a discharge orifice (1342), the suction orifice (1341) being disposed on the cantilever branch (133), the discharge orifice (1342) being disposed on the bearing branch (132).

7. The dental stabilization block (100) according to claim 6, wherein the bearing branch (132) comprising a suction cannula guide part (1321) which is open in the direction of the second side wall (114) and which extends axially from the discharge orifice (1342) to the free end of the bearing branch (132) .

8. The dental stabilization block (100) according to any one of claims 1 to 7, wherein the upper outer tightening surface and the lower outer tightening surface are provided with a plurality of fluid flow disturbance ribs (1112).

9. The dental stabilization block (100) according to any one of claims 5 to 7, further comprising an extension branch (140) which extends obliquely or perpendicularly from the free end of the bearing branch (132) towards the outside of the mouth, the extension branch (140) being designed to form a direction change region (141) which, when the extension branch (140) deflects to come out of the patient's mouth, has an external surface that covers the labial commissure of the mouth,
wherein,
at least one free end region of the extension branch (140) comprises at least one support element (142, 143) which is adapted to support at least one position reference element which is designed to serve as a guide element for a navigation system for dental surgery, the position reference element being adapted, when the position reference element is moved in response to at least one movement of the patient's head, to trigger a registration of one or several dental images associated with the patient, based on the movement of the patient's head.

10. The dental stabilization system comprising:
- a dental stabilization block (100) according to claim 9, and
- a position reference element which is configured to be coupled to at least one support element (142, 143) of the free end region of the extension branch (140), the position reference element being designed to serve as a guide element for a navigation system for dental surgery, the position reference element being adapted, when the position reference element is moved in response to at least one movement of the patient's head, to trigger a registration of one or several dental images associated with the patient, based on the movement of the patient's head.

11. The dental stabilization system comprising:
- two dental stabilization blocks (100) according to any one of claims 1 to 9, the dental stabilization blocks (100) being configured to be positioned on opposite sides of the patient's mouth.

12. A method (200) for dental stabilization during a dental procedure, the method comprising:
- a step of installing (210), in the patient's mouth, at least one dental stabilization block (100) according to any one of claims 1 to 9, on upper and lower molars and/or premolars of a patient, called teeth,
- a step of introducing (220) an end portion of a device for injecting a bite registration material to the inlet orifice (1131), the bite registration material being intended to harden and to be molded at least on the teeth, and
- a step of injecting (230) the bite registration material into the inlet orifice (1131).
